(19) **Europäisches Patentamt / European Patent Office / Office européen des brevets**

(11) **EP 2 780 853 B1**

(12) **EUROPEAN PATENT SPECIFICATION**

(45) Date of publication and mention of the grant of the patent:
**01.05.2019 Bulletin 2019/18**

(21) Application number: **12798036.5**

(22) Date of filing: **28.09.2012**

(51) Int Cl.:
***G06F 19/24*** (2011.01)   ***G06F 19/26*** (2011.01)

(86) International application number:
**PCT/US2012/057707**

(87) International publication number:
**WO 2013/074204 (23.05.2013 Gazette 2013/21)**

(54) **METHOD AND SYSTEM FOR DETERMINING AN AMPLIFICATION QUALITY METRIC**

VERFAHREN UND SYSTEM ZUR BESTIMMUNG EINER AMPLIFIKATIONSQUALITÄTSMASSZAHL

PROCÉDÉ ET SYSTÈME PERMETTANT DE DÉTERMINER UNE MÉTRIQUE DE QUALITÉ D'AMPLIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2011 US 201161559636 P**

(43) Date of publication of application:
**24.09.2014 Bulletin 2014/39**

(73) Proprietor: **Life Technologies Corporation Carlsbad, CA 92008 (US)**

(72) Inventors:
• **WEISS, Howard**
  **Carlsbad, California 92008 (US)**
• **VAN HELEN, Eric**
  **Carlsbad, California 92008 (US)**
• **CHO, Jamie**
  **Carlsbad, California 92008 (US)**
• **WESSEL, Thomas**
  **Carlsbad, California 92008 (US)**
• **MORRISON, Thomas B.**
  **Wilmington, North Carolina 28409 (US)**

(74) Representative: **Hoffmann Eitle Patent- und Rechtsanwälte PartmbB Arabellastraße 30 81925 München (DE)**

(56) References cited:
EP-A2- 1 041 158       WO-A1-2006/048337
WO-A1-2010/025985    US-A1- 2006 265 143

## Description

BACKGROUND

Polymerase Chain Reaction (PCR) instrumentation has made it possible to perform reliable quantification of DNA or RNA levels in biological samples. Commercially available PCR instruments, and related data acquisition and analysis software, process qPCR assay data generated from biological samples. These systems report quantitative results by calculating a threshold cycle (CT or CRT) value as the fractional PCR cycle number where the reporter signal rises above a threshold set manually by a human or automatically by software. The determined CT value can be used to estimate the initial quantity of DNA material.

In amplifying a plurality of samples, many samples may have weak or non-amplifications. In a large amount of data, it is challenging to determine which samples may have weak or non-amplifications. In other words, the data generated from these samples may not be as reliable or produce accurate results.

SUMMARY

The present invention provides a method for providing an amplification quality metric to a user with the features of claim 1, a computer-readable medium encoded with instructions with the features of claim 5 and a system for providing an amplification quality metric to a user with the features of claim 9.

DESCRIPTION OF THE FIGURES

FIG. 1A illustrates a block diagram of an exemplary computing system according to various embodiments described herein;

FIG. 2 illustrates a block diagram of an exemplary thermal cycler system according to various embodiments described herein;

FIG. 3A illustrates an exemplary strong amplification curve;

FIG. 3B illustrates an exemplary weak amplification curve;

FIG. 3C illustrates an exemplary non-amplification curve;

FIG. 4 illustrates an example of raw data of an amplification of sample;

FIG. 5A illustrates an exemplary amplification curve smoothed according to various embodiments described herein;

FIG. 5B illustrates an exemplary amplification curve according to various embodiments described herein;

FIG. 6 illustrates results determined by the amplification quality metric according to various embodiments described herein; and

FIG. 7 illustrates a comparison of an exemplary confidence value with an amplification quality metric according to various embodiments described herein;

FIG. 8 illustrates another comparison of an exemplary confidence value with an amplification quality metric according to various embodiments described herein;

FIG. 9 illustrates results determined by the amplification quality metric according to various embodiments described herein;

FIG. 10 illustrates a plot generated by filtering based on amplification quality metrics according to various embodiments described herein; and

FIG. 11 illustrates a plot generated by filtering based on amplification quality metrics according to various embodiments described herein.

DETAILED DESCRIPTION

To provide a more thorough understanding of the present invention, the following description sets forth numerous specific details, such as specific configurations, parameters, examples, and the like. It should be recognized, however, that such description is not intended as a limitation on the scope of the present invention, but is intended to provide a better description of the exemplary embodiments.

As described above, a current challenge is determining reliable data in sample amplification. As such, an amplification quality metric that will provide a user-visible metric of reaction quality is desired. Additionally, an amplification quality metric to distinguish amplified from non-amplified reactions and a consistent confidence metric for classifying a large number of samples are desired. Further, a confidence metric that can span a large range of cycles, increasing the dynamic range, is also desired.

In various embodiments, the devices, instruments, systems, and methods described herein may be used to detect one or more types of biological components of interest. These biological components of interest may be any suitable biological target including, but are not limited to, DNA sequences (including cell-free DNA), RNA sequences, genes, oligonucleotides, molecules, proteins, biomarkers, cells (e.g., circulating tumor cells), or any other suitable target biomolecule.

In various embodiments, such biological components may be used in conjunction with various PCR, qPCR, and/or dPCR methods and systems in applications such as fetal diagnostics, multiplex dPCR, viral detection and quantification standards, genotyping, sequencing validation, mutation detection, detection of genetically modified organisms, rare allele detection, and copy number variation. Embodiments of the present disclosure are generally directed to devices, instruments, systems, and methods for monitoring or measuring a biological reaction for a large number of small volume samples. As used herein, samples may be referred to as sample volumes, or reactions volumes, for example..

While generally applicable to quantitative polymerase chain reactions (qPCR) where a large number of samples are being processed, it should be recognized that any suitable PCR method may be used in accordance with various embodiments described herein. Suitable PCR methods include, but are not limited to, digital PCR, allele-specific PCR, asymmetric PCR, ligation-mediated PCR, multiplex PCR, nested PCR, qPCR, genome walking, and bridge PCR, for example.

As described below, in accordance with various embodiments described herein, reaction sites may include, but are not limited to, through-holes, wells, indentations, spots, cavities, sample retainment regions, and reaction chambers, for example.

Furthermore, as used herein, thermal cycling may include using a thermal cycler, isothermal amplification, thermal convention, infrared mediated thermal cycling, or helicase dependent amplification, for example. In some embodiments, the chip may be integrated with a built-in heating element. In various embodiments, the chip may be integrated with semiconductors.

According to various embodiments, detection of a target may be, but is not limited to, fluorescence detection, detection of positive or negative ions, pH detection, voltage detection, or current detection, alone or in combination, for example.

Various embodiments described herein are particularly suited for digital PCR (dPCR). In digital PCR, a solution containing a relatively small number of a target polynucleotide or nucleotide sequence may be subdivided into a large number of small test samples, such that each sample generally contains either one molecule of the target nucleotide sequence or none of the target nucleotide sequence. When the samples are subsequently thermally cycled in a PCR protocol, procedure, or experiment, the sample containing the target nucleotide sequence are amplified and produce a positive detection signal, while the samples containing no target nucleotide sequence are not amplified and produce no detection signal. Using Poisson statistics, the number of target nucleotide sequences in the original solution may be correlated to the number of samples producing a positive detection signal. Positive and negative detection can be determined or validated by amplification quality metrics according to various embodiments of the present teachings.

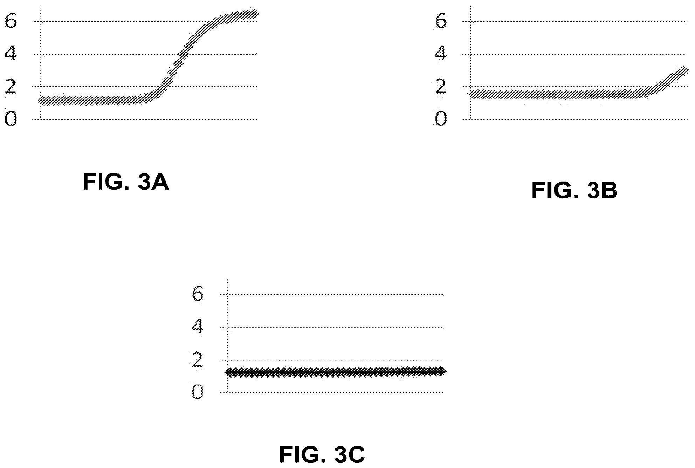

According to the embodiments described herein, an amplification quality metric may be determined by using the height of the transition region, which correlates with reaction quality. A high rise in the transition region implies strong amplification. An example of an amplification curve of a strong amplification is illustrated in FIG. 3A. A lower rise in the transition region implies weak amplification. An example of an amplification curve of a weak amplification is illustrated in FIG. 3B. Non-existent rise in the linear region implies non-amplification. An example of an amplification curve of a non-amplification is shown in FIG. 3C. According to various embodiments described herein, an amplification quality metric or metric that measures the rise in the transition region so as to be able to distinguish amplified from non-amplified curves is provided.

According to various embodiments described herein, an amplification quality metric may indicate a morphology, or shape, of an amplification curve that may give more information about the nature of amplification or non-amplification of a sample. For example, the amplification quality metric may help in determining a plateau region of the amplification curve. Further, amplification quality metrics may help a user identity and filter amplification curves especially when an experiment has a plurality of samples. Data of the plurality of samples may be more easily visualized by a user. The ability to filter and visualize amplification curves may help a user troubleshoot a system.

Amplification can be visualized by amplification curves generated by detecting fluorescence. Amplification Curves are typically described as consisting of 4 separate regions: baseline, exponential, transition (or linear), and plateau. The baseline region is the region where the signal dominated by background. The exponential region is the region where the signal dominated by double of product at each cycle. The transition region, also known as the linear region, is the region of the curve where the signal is no longer doubling as efficiency drops significantly. Finally, the plateau region is where the signal is no longer growing due to depleted reagents.

Those skilled in the art will recognize that the operations of the various embodiments may be implemented using hardware, software, firmware, or combinations thereof, as appropriate. For example, some processes can be carried out using processors or other digital circuitry under the control of software, firmware, or hardwired logic. (The term "logic" herein refers to fixed hardware, programmable logic and/or an appropriate combination thereof, as would be recognized by one skilled in the art to carry out the recited functions.) Software and firmware can be stored on computer-readable media. Some other processes can be implemented using analog circuitry, as is well known to one of ordinary skill in the art. Additionally, memory or other storage, as well as communication components, may be employed in embodiments of the invention.

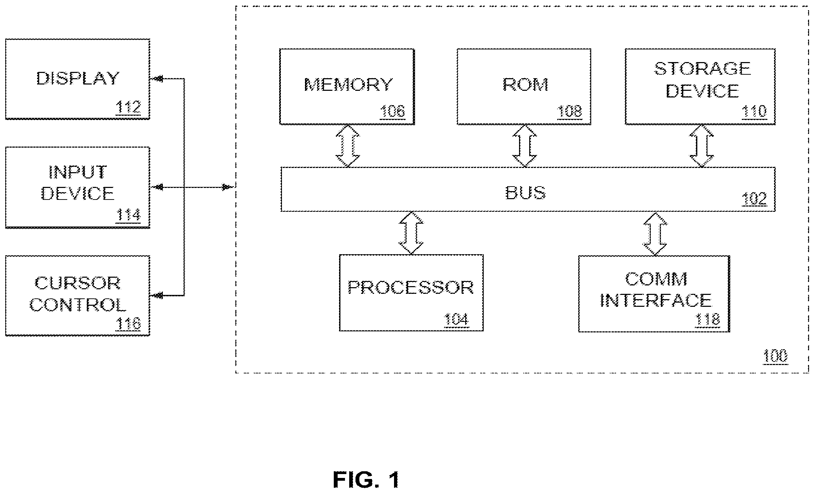

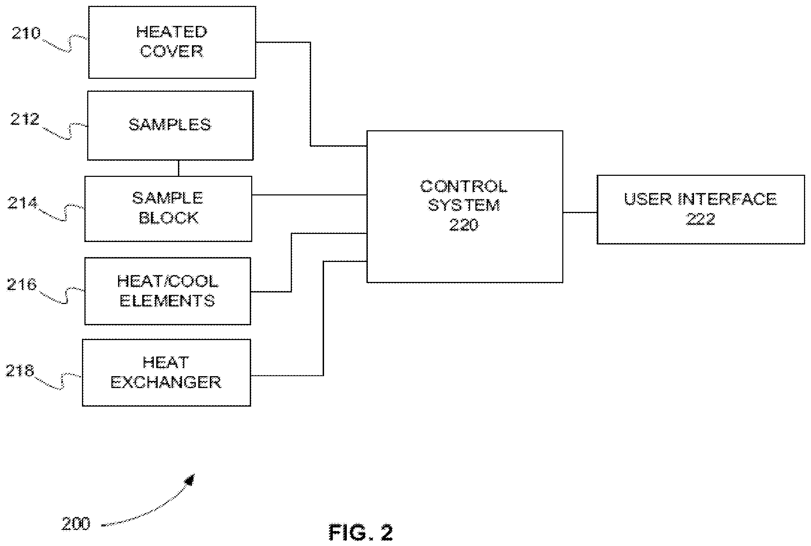

FIG. 1 is a block diagram that illustrates a computer system 100 that may be employed to carry out processing functionality, according to various embodiments, upon which embodiments of a thermal cycler system 200 of FIG. 2 may utilize. Computing system 100 can include one or more processors, such as a processor 104. Processor 104 can be implemented using a general or special purpose processing engine such as, for example, a microprocessor, controller or other control logic. In this example, processor 104 is connected to a bus 102 or other communication medium.

Further, it should be appreciated that a computing system 100 of FIG. 1 may be embodied in any of a number of forms, such as a rack-mounted computer, mainframe, supercomputer, server, client, a desktop computer, a laptop computer, a tablet computer, handheld computing device (e.g., PDA, cell phone, smart phone, palmtop, etc.), cluster grid, netbook, embedded systems, or any other type of special or general purpose computing device as may be desirable or appropriate for a given application or environment. Additionally, a computing system 100 can include a conventional network system including a client/server environment and one or more database servers, or integration with LIS/LIMS infrastructure. A number of conventional network systems, including a local area network (LAN) or a wide area network (WAN), and including wireless and/or wired components, are known in the art. Additionally, client/server environments, database servers, and networks are well documented in the art.

Computing system 100 may include bus 102 or other communication mechanism for communicating information, and processor 104 coupled with bus 102 for processing information.

Computing system 100 also includes a memory 106, which can be a random access memory (RAM) or other dynamic memory, coupled to bus 102 for storing instructions to be executed by processor 104. Memory 106 also may be used for storing temporary variables or other intermediate information during execution of instructions to be executed by processor 104. Computing system 100 further includes a read only memory (ROM) 108 or other static storage device coupled to bus 102 for storing static information and instructions for processor 104.

Computing system 100 may also include a storage device 110, such as a magnetic disk, optical disk, or solid state drive (SSD) is provided and coupled to bus 102 for storing information and instructions. Storage device 110 may include a media drive and a removable storage interface. A media drive may include a drive or other mechanism to support fixed or removable storage media, such as a hard disk drive, a floppy disk drive, a magnetic tape drive, an optical disk drive, a CD or DVD drive (R or RW), flash drive, or other removable or fixed media drive. As these examples illustrate, the storage media may include a computer-readable storage medium having stored therein particular computer software, instructions, or data.

In alternative embodiments, storage device 110 may include other similar instrumentalities for allowing computer programs or other instructions or data to be loaded into computing system 100. Such instrumentalities may include, for example, a removable storage unit and an interface, such as a program cartridge and cartridge interface, a removable memory (for example, a flash memory or other removable memory module) and memory slot, and other removable storage units and interfaces that allow software and data to be transferred from the storage device 110 to computing system 100.

Computing system 100 can also include a communications interface 118. Communications interface 118 can be used to allow software and data to be transferred between computing system 100 and external devices. Examples of communications interface 118 can include a modem, a network interface (such as an Ethernet or other NIC card), a communications port (such as for example, a USB port, a RS-232C serial port), a PCMCIA slot and card, Bluetooth, etc. Software and data transferred via communications interface 118 are in the form of signals which can be electronic, electromagnetic, optical or other signals capable of being received by communications interface 118. These signals may be transmitted and received by communications interface 118 via a channel such as a wireless medium, wire or cable, fiber optics, or other communications medium. Some examples of a channel include a phone line, a cellular phone link, an RF link, a network interface, a local or wide area network, and other communications channels.

Computing system 100 may be coupled via bus 102 to a display 112, such as a cathode ray tube (CRT) or liquid crystal display (LCD), for displaying information to a computer user. An input device 114, including alphanumeric and other keys, is coupled to bus 102 for communicating information and command selections to processor 104, for example. An input device may also be a display, such as an LCD display, configured with touchscreen input capabilities. Another type of user input device is cursor control 116, such as a mouse, a trackball or cursor direction keys for communicating direction information and command selections to processor 104 and for controlling cursor movement on display 112. This input device typically has two degrees of freedom in two axes, a first axis (e.g., x) and a second axis (e.g., y), that allows the device to specify positions in a plane. A computing system 100 provides data processing and provides a level of confidence for such data. Consistent with certain implementations of embodiments of the present teachings, data processing and confidence values are provided by computing system 100 in response to processor 104 executing one or more sequences of one or more instructions contained in memory 106. Such instructions may be read into memory 106 from another computer-readable medium, such as storage device 110. Execution of the sequences of instructions contained in memory 106 causes processor 104 to perform the process states described herein. Alternatively hard-wired circuitry may be used in place of or in combination with software instructions to implement embodiments of the present teachings. Thus implementations of embodiments of the present teachings are not limited to any specific combination of hardware circuitry and software.

The term "computer-readable medium" and "computer program product" as used herein generally refers to any media that is involved in providing one or more sequences or one or more instructions to processor 104 for execution. Such instructions, generally referred to as "computer program code" (which may be grouped in the form of computer programs or other groupings), when executed, enable the computing system 100 to perform features or functions of embodiments of the present invention. These and other forms of computer-readable media may take many forms, including but not limited to, non-volatile media, volatile media, and transmission media. Non-volatile media includes, for example, solid state, optical or magnetic disks, such as storage device 110. Volatile media includes dynamic memory, such as memory 106. Transmission media includes coaxial cables, copper wire, and fiber optics, including the wires that comprise bus 102.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, a carrier wave as described hereinafter, or any other medium from which a computer can read.

Various forms of computer readable media may be involved in carrying one or more sequences of one or more instructions to processor 1 04 for execution. For example, the instructions may initially be carried on magnetic disk of a remote computer. The remote computer can load the instructions into its dynamic memory and send the instructions over a telephone line using a modem. A modem local to computing system 100 can receive the data on the telephone line and use an infra-red transmitter to convert the data to an infra-red signal. An infra-red detector coupled to bus 102 can receive the data carried in the infra-red signal and place the data on bus 102. Bus 102 carries the data to memory 106, from which processor 104 retrieves and executes the instructions. The instructions received by memory 106 may optionally be stored on storage device 110 either before or after execution by processor 104.

It will be appreciated that, for clarity purposes, the above description has described embodiments of the invention with reference to different functional units and processors. However, it will be apparent that any suitable distribution of functionality between different functional units, processors or domains may be used without detracting from the invention. For example, functionality illustrated to be performed by separate processors or controllers may be performed by the same processor or controller. Hence, references to specific functional units are only to be seen as references to suitable means for providing the described functionality, rather than indicative of a strict logical or physical structure or organization.

To prepare, observe, test, and/or analyze an array of biological samples, one example of an instrument that may be utilized according to various embodiments is a thermal cycler device, such as an end-point polymerase chain reaction (PCR) instrument or a quantitative, or real-time, PCR instrument. FIG. 2 is a block diagram that illustrates a thermal cycler 200, upon which embodiments of the present teachings may be implemented. Thermal cycler 200 may include a heated cover 210, discussed in greater detail below, which is placed over a sample block 214 loaded with a plurality of samples 212 contained in a sample holder (not shown), also discussed in greater detail below.

In various embodiments, the sample holder may have a plurality of sample regions, or reaction sites, configured for receiving a plurality of samples, wherein the reaction sites may be sealed within the sample holder via a lid between the reaction sites and heated cover 210. Some examples of a sample holder may include, but are not limited to, a multi-well plate, such as a standard microtiter 96-well plate, a 384-well plate, a microcard, a through-hole array, or a substantially planar holder, such as a glass or plastic slide. The reaction sites in various embodiments of a sample holder may include depressions, indentations, ridges, and combinations thereof, patterned in regular or irregular arrays formed on the surface of the sample holder substrate.

According to various embodiments of the present teachings, each reaction sites may have a volume of about 1.3 nanoliters. Alternatively, the volume each reaction site may be less than 1,3 nanoliters. This may be achieved, for example, by decreasing the diameter of reaction site 104 and/or the thickness of the sample holder. For example, each reaction site 104 may have a volume that is less than or equal to 1 nanoliter, less than or equal to 100 picoliters, less than or equal to 30 picoliters, or less than or equal to 10 picoliters. In other embodiments, the volume some or all of the reaction sites 104 is in a range of 1 to 20 nanoliters.

In some embodiments, the reaction sites are through-holes. In these examples, each through-hole has a volume of about 1.3 nanoliters. Alternatively, the volume each through-hole may be less than 1.3 nanoliters. This may be achieved, for example, by decreasing the diameter of through-hole and/or the thickness of the sample holder substrate. For example, each through-hole may have a volume that is less than or equal to 1 nanoliter, less than or equal to 100 picoliters, less than or equal to 30 picoliters, or less than or equal to 10 picoliters. In other embodiments, the volume some or all of the through-holes is in a range of 1 to 20 nanoliters.

In various embodiments, a density of reaction sites 104 may be at least 100 reaction sites per square millimeter. In other embodiments, there may be higher densities of reaction sites. For example, a density of reaction sites 104 within chip 100 may be greater than or equal to 150 reaction sites per square millimeter, greater than or equal to 200 reaction sites per square millimeter, greater than or equal to 500 reaction sites per square millimeter, greater than or equal to 1,000 reaction sites per square millimeter, greater than or equal to 10,000 reaction sites per square millimeter.

In some embodiments, the reaction sites are through-holes. Accordingly, a density of through-holes within a sample holder substrate may be greater than or equal to 150 through-holes per square millimeter, greater than or equal to 200 through-holes per square millimeter, greater than or equal to 500 through-holes per square millimeter, greater than or equal to 1,000 through-holes per square millimeter, greater than or equal to 10,000 through-holes per square millimeter.

Thermal cycler 200 may also include a sample block 214, elements for heating and cooling 216, a heat exchanger 218, a control system 220, and a user interface 222, wherein components 214, 216 and 218 can be included within a thermal block assembly. The thermal block assembly can have an interchangeable feature such that thermal block assembly can be configured to accommodate any one of the multiple sample holders, and their associated sample blocks, stated above.

Additionally, various embodiments of a thermal cycling system 200 may have a detection system (not shown). A detection system may have an illumination source that emits electromagnetic energy (not shown), a detector or imager (not shown), for receiving electromagnetic energy from samples 212 in sample support device, and optics (not shown), which may be located between the illumination source and detector or imager (not shown). For various embodiments of a thermal cycler instrument 200, a control system 220 may be used to control, for example, but not limited by, the functions of the detection, heated cover, and thermal block assembly. The control system 220 may be accessible to an end user through user interface 222 of a thermal cycler instrument 200.

According to various embodiments described herein, a amplification quality metric is calculated in four main stages, each of which is described below.

### Amplification Curve Filtering

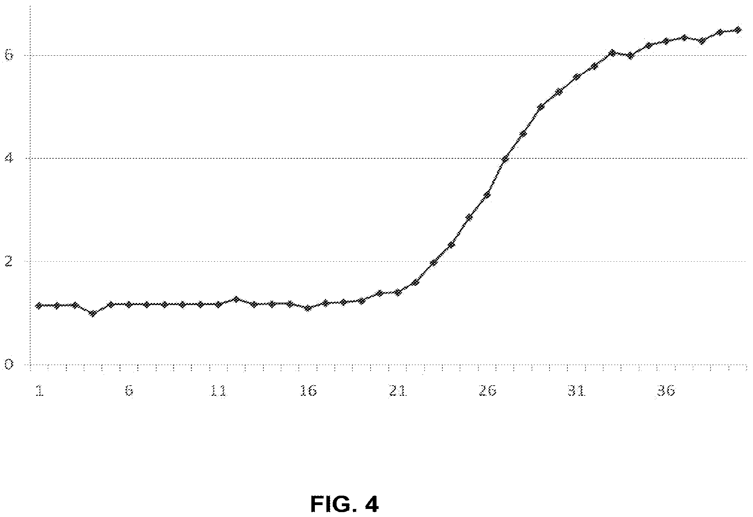

In the first stage, according to various embodiments, the amplification curve data is filtered to smooth out local variability. A raw data amplification curve is shown in FIG. 4. In other words, according to various embodiments, the amplification is smoothed out to reduce variation due to random noise in the amplification curve on the order of less than 5 cycles.

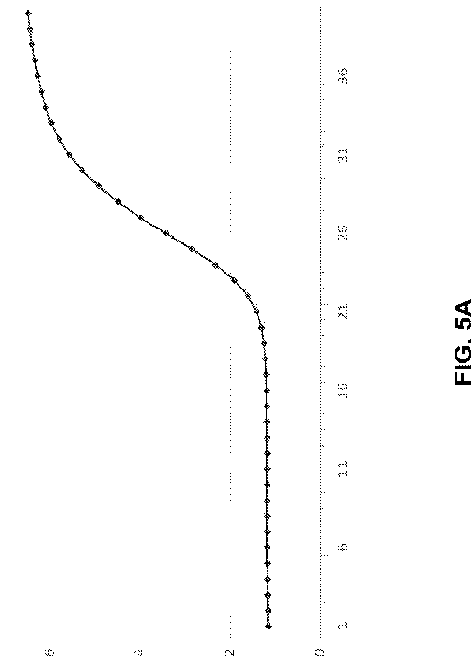

FIG. 5A illustrates an exemplary smoothed amplification curve 500 according to embodiments described in this document.

According to an exemplary embodiment, amplification curve filtering is accomplished using a 7 point, 2nd degree Savitzky-Golay smoothing filter. However, it should be recognized that other filters may be used to smooth the amplification curve to reduce random noise in the curve.

### Beginning of Transition Region Identification

In the next stage, according to various embodiments, a first value is found corresponding to the start of the transition region. The transition region, as described above, is the region of interest in determining the amplification quality metric according to various embodiments. The start of the transition regions is identified as the region directly follow the end of the exponential region and directly prior to the start of the plateau region.

In some embodiments, the transition region may be identified by identifying the end of the exponential region. As such, according to various embodiments, the beginning of the transition region may be determined by computing the peak of the second derivative of the amplification curve proper. In other words, the maximum value of the second derivative of the amplification curve may be the first value used to calculate the amplification quality metric according to embodiments described herein.

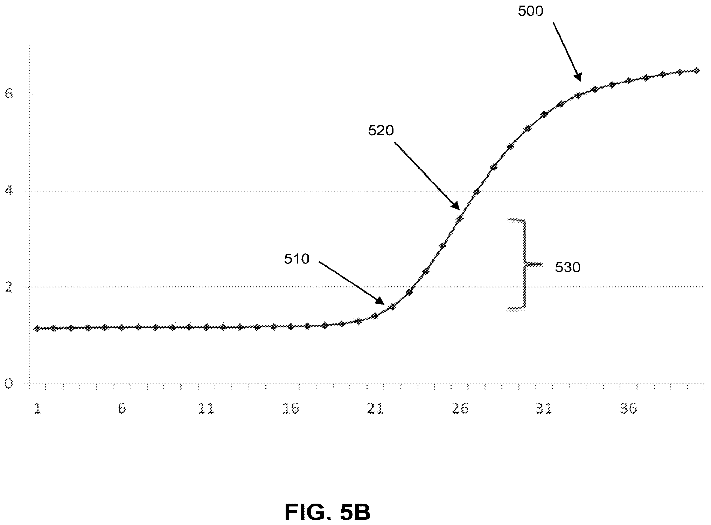

FIG. 5B illustrates the beginning 510 and the end 520 of the exponential region. The difference between the value at the end 520 of the exponential region and the value at the beginning 510 of the exponential region is denoted by reference sign 530.

### End of Transition Region Identification

According to embodiments described herein, an upper second value is determined to calculate the amplification quality metric. The second value identifies the end of the transition region. The difference between the second value and the first value results in the raw amplification quality metric.

In other words, the raw amplification quality metric is the difference between the value at the end of the transition region (ETRI) and the value at the beginning of the transition region (BTRI), represented as follows:

$$S = F(ETRI) - F(BTRI)$$

According to one exemplary embodiment, the transition region of the amplification curve is transversed four cycles to determine the second value. The fluorescence value at this point four cycles from the beginning of the transition region is established as the upper value used in calculating the amplification quality metric according to various embodiments.

However, in some embodiments, if the transition region is identified as being within four cycles of the end of the amplification, then the fluorescence value at the end of the amplification curve is established as the second value. It should be recognized that four cycles is not necessarily the only number of cycles that could be used in determining the second value. Based on empirical data, potential other values could be, but are not limited to, two to five, for example.

**Amplification quality metric Normalization**

According to various embodiments, the raw amplification quality metric is normalized. Normalization may accounts for the presence or absence of passive references. For example, in amplification curves generated with no passive reference, $\Delta R_n$s can be in the thousands and amplification quality metrics may range from zero to multiple thousands. In TAQMAN amplification curves (with ROX normalization) $\Delta R_n$s may be range from fractions to double digits and amplification quality metrics may range from zero to double digits. By normalization, a single amplification quality metric can accommodate both types of data.

As such, the amplification curve may be normalized in the following manner depending on the presence or absence of passive reference normalization, according to some embodiments:

$$\hat{S} = \frac{\log_{10}(S)}{2},$$

absent passive reference normalization of the fluorescence curve

$$\hat{S} = \frac{\log_{10}(500 * S)}{2},$$

with passive reference normalization of the fluorescence curve.

According to various embodiments, normalization of the amplification quality metric translates to having an initial raw amplification quality metric of 100 (when passive reference normalization is not present) and an initial raw amplification quality metric of 0.2 (when passive reference normalization is present) equal a value of 1.0 for the normalized amplification quality metric. Empirically, these initial raw scores (100 for non-passive reference and 0.2 for TaqMan passive reference) were found to successfully distinguish between annotated amplified versus non-amplified curves.

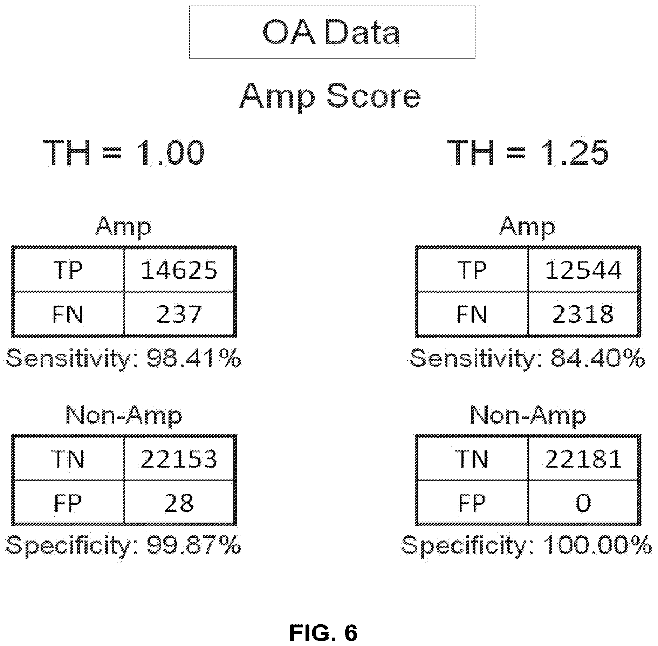

Some results to illustrate the accuracy of the amplification quality metric according to various embodiments are shown in FIGs. 6-9. In some embodiments, amplification quality metrics less than 1 were correlated with non-amplifications. Amplification quality metrics greater than 1 were correlated with amplifications. In other embodiments, a different threshold for amplification quality metrics to determine amplifications from non-amplifications may be used. For example, 1.25 may be used as the threshold, as shown in the exemplary results in FIG. 6.

FIG. 6 shows the resulting positive amplifications and non-amplification determinations of a data set compared to manual classification of the same data set for two different thresholds of the amplification quality metric according to various embodiments. The data set included 38,393 amplification curves. In the manual classification, 22,181 curves were determined to be non-amplification curves, 14,862 were determined to be amplified amplification curves, and 1,350 were determined to be indeterminate.

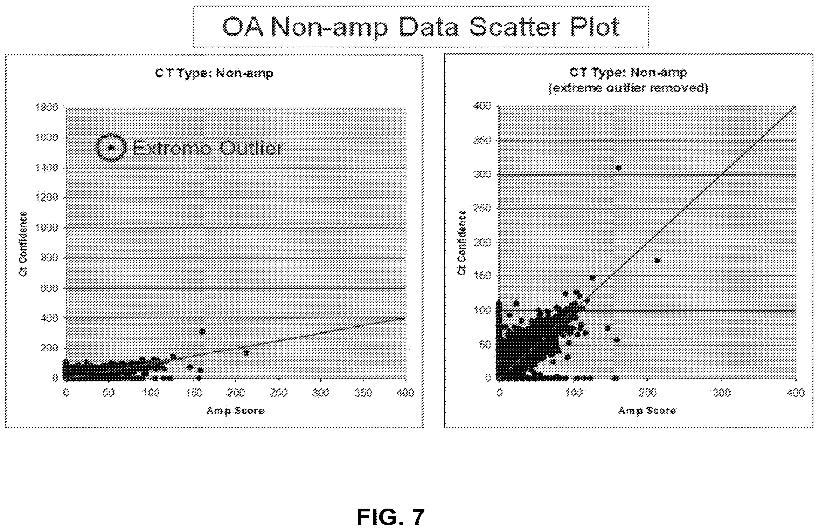

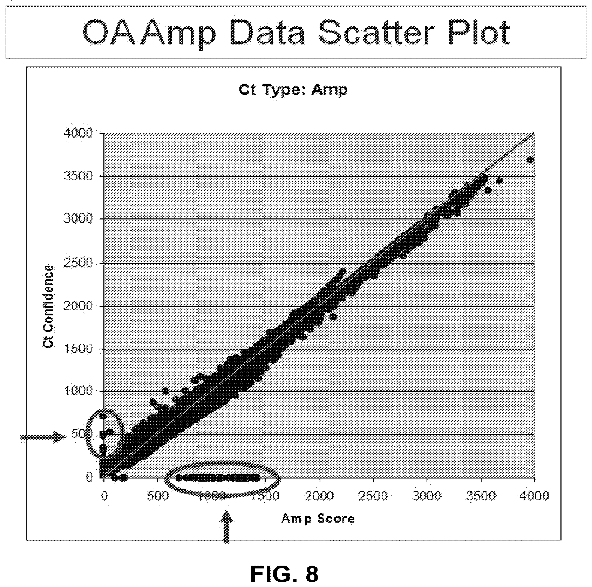

FIGs. 7 and 8 illustrate scatter plots of the amplifications and non-amplifications determined by using confidence values and amplification quality metrics according to various embodiments.

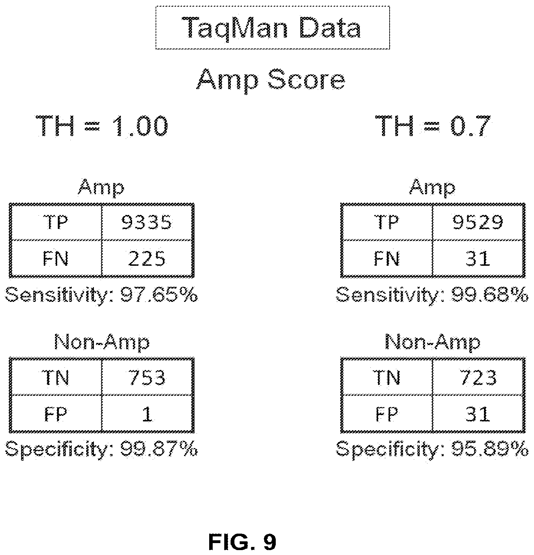

FIG. 9 shows the resulting positive amplifications and non-amplification determinations of a TAQMAN data set compared to manual classification of the same data set for two different thresholds of the amplification quality metric according to various embodiments.

The TAQMAN data set included 10,368 amplification curves. In the manual classification, 754 curves were determined to be non-amplification curves, 9,560 were determined to be amplified amplification curves, and 54 were determined to be indeterminate. Both FAM and SYBR data were included. In other embodiments, a passive reference is not needed in determining an amplification quality metric.

According to various embodiments, amplification curves may be filtered by setting an amplification quality metric threshold. In this way, a user may be able to better determine which samples of the plurality of samples did not amplify. Alternatively, a user may desire to see which samples of the plurality of sample did amplify. As such, the amplification curves that exceed or meet an amplification quality metric are displayed on a display. An amplification quality metric threshold may be automatically determined in some embodiments. In other embodiments, a user may define the amplification quality metric threshold.

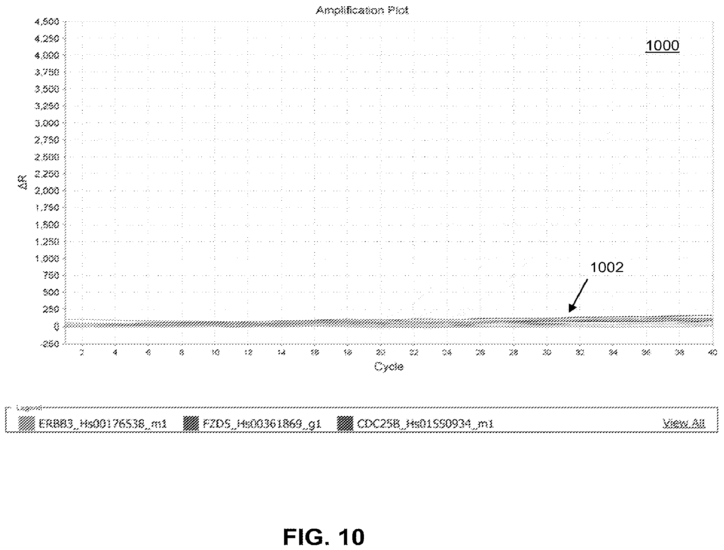

FIG. 10 illustrates a visualization that may be generated by filtering amplification curves based on amplification quality metrics according to embodiments described herein. The visualization may be provided to a user on a display so that the user may visualize the amplification of the plurality of samples within the plurality of reaction sites. Plot 1000 shows a plurality of amplification curves 1002 below an amplification quality metric threshold of 0.9. As such, a user is able to more easily identify which samples did not amplify. According to some embodiments, being able to identify specific reactions sites with samples that did not amplify could help to troubleshoot the experiment.

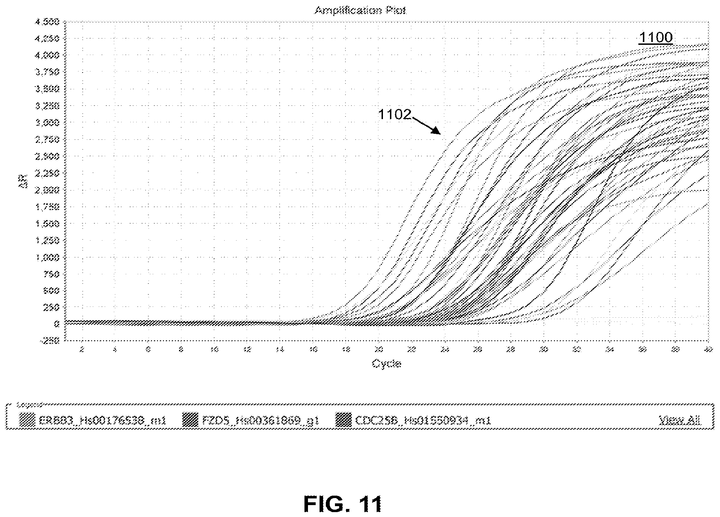

FIG. 11 illustrates a visualization that may be generated by filtering amplification curves based on amplification quality metrics according to embodiments described herein. Plot 1100 illustrates a plurality of amplification curves 1102 that exceed an amplification quality metric threshold of 1.4. Thus, a user may be able to identify which samples amplified.

According to various embodiments described herein, a method for providing a amplification quality metric to a user is provided. The method may be implemented by a processor or computing system as described above.

Therefore, according to the above, some examples of the disclosure comprise the following:

In one example, the method includes receiving amplification data from an amplification of a sample to generate an amplification curve, where the amplification curve includes an exponential region and a transition region. The method further includes determining a first value of the transition region, wherein the first value is the beginning of the transition region and determining a second value of the transition region, wherein the second value is the end of the transition region. The method further includes calculating a amplification quality metric based on at least the first value and the second value and displaying the amplification quality metric to a user.

Additionally or alternatively, in one or more of the examples disclosed above, the amplification quality metric of each of the plurality of amplification curves may be used to filter the amplification curves based on an amplification quality metric threshold to display to the user.

Additionally or alternatively, in one or more of the examples disclosed above, the method may further include filtering the amplification data. The method may further include normalizing the calculated amplification quality metric.

Additionally or alternatively, to one or more of the examples disclosed above, determining the first value of the transition region may comprise calculating the second derivative of the amplification curve, wherein the peak of a maximum of the second derivative is the first value. Additionally or alternatively, to one or more of the examples disclosed above, determining the second value of the transition region may comprise determining a value of the amplification curve 2-5 cycles from the first value.

Additionally or alternatively, in one or more of the examples disclosed above, determining the second value of the transition region comprises determining a value of the amplification curve 4 cycles from the first value.

Additionally or alternatively, in one or more of the examples disclosed above, calculating the amplification quality metric is taking the difference of the first value and the second value.

In another example, a computer-readable medium encoded with instructions, executable by a processor, is provided. The instructions include instructions for determining a first value of the transition region, where the first value is the beginning of the transition region. The instructions further include instructions for determining a second value of the transition region, wherein the second value is the end of the transition region, calculating a amplification quality metric based on at least the first value and the second value, and displaying the amplification quality metric to a user.

Additionally or alternatively, in one or more of the examples disclosed above, the computer-readable medium further comprises instructions for filtering the amplification data.

Additionally or alternatively, in one or more of the examples disclosed above, the computer-readable medium further comprises instructions for normalizing the calculated amplification quality metric.

Additionally or alternatively, in one or more of the examples disclosed above, the amplification quality metric of each of the plurality of amplification curves may be used to filter the amplification curves based on an amplification quality metric threshold to display to the user.

Additionally or alternatively, in one or more of the examples disclosed above, the method may further include filtering the amplification data. The method may further include normalizing the calculated amplification quality metric.

Additionally or alternatively, to one or more of the examples disclosed above, determining the first value of the transition region may comprise calculating the second derivative of the amplification curve, wherein the peak of a maximum of the second derivative is the first value. Additionally or alternatively, to one or more of the examples disclosed above, determining the second value of the transition region may comprise determining a value of the amplification curve 2-5 cycles from the first value.

Additionally or alternatively, in one or more of the examples disclosed above, determining the second value of the transition region comprises determining a value of the amplification curve 4 cycles from the first value.

Additionally or alternatively, in one or more of the examples disclosed above, calculating the amplification quality metric is taking the difference of the first value and the second value.

In another example, a system for providing a amplification quality metric to a user is provided. The system comprises a processor and a memory for storing instructions executable by the processor. The instructions comprising instructions for: receiving amplification data from an amplification of a sample to generate an amplification curve, wherein the amplification curve includes an exponential region and a transition region; determining a first value of the transition region, wherein the first value is the beginning of the transition region; determining a second value of the transition region, wherein the second value is the end of the transition region; calculating a amplification quality metric based on at least the first value and the second value; and displaying the amplification quality metric to a user.

Additionally or alternatively, in one or more of the examples disclosed above, the instructions further comprise instructions for filtering the amplification data.

Additionally or alternatively, in one or more of the examples disclosed above, the amplification quality metric of each of the plurality of amplification curves may be used to filter the amplification curves based on an amplification quality metric threshold to display to the user.

Additionally or alternatively, in one or more of the examples disclosed above, the method may further include filtering the amplification data. The method may further include normalizing the calculated amplification quality metric.

Additionally or alternatively, to one or more of the examples disclosed above, determining the first value of the transition region may comprise calculating the second derivative of the amplification curve, wherein the peak of a maximum of the second derivative is the first value. Additionally or alternatively, to one or more of the examples disclosed above, determining the second value of the transition region may comprise determining a value of the amplification curve 2-5 cycles from the first value.

Additionally or alternatively, in one or more of the examples disclosed above, determining the second value of the transition region comprises determining a value of the amplification curve 4 cycles from the first value.

Additionally or alternatively, in one or more of the examples disclosed above, calculating the amplification quality metric is taking the difference of the first value and the second value.

Although the present invention has been described with respect to certain exemplary embodiments, examples, and applications, it will be apparent to those skilled in the art that various modifications and changes may be made without departing from the invention.

**Claims**

1. A computer-implemented method for providing an amplification quality metric to a user, the method comprising:

     receiving amplification data from a PCR amplification of a sample (212) to generate a PCR amplification curve (500), wherein the amplification curve (500) includes an exponential region and a transition region;
     determining a first value of the transition region, wherein the first value is a value of the amplification curve at the beginning of the transition region;
     determining a second value of the transition region, wherein the second value is a value of the amplification curve at the end of the transition region;
     calculating an amplification quality metric based on at least the first value and the second value; and

     displaying the amplification quality metric to a user;
     wherein the beginning of the transition region is the end of the exponential region,
     wherein determining the second value of the transition region comprises determining a value of the amplification curve 2-5 cycles from the first value,
     wherein the beginning of the transition region is determined by computing the peak of the second derivative of the amplification curve (500), and
     wherein calculating the amplification quality metric is taking the difference of the first value and the second value.

2. The method of claim 1, further comprising filtering the amplification data.

3. The method of claim 1, wherein determining the second value of the transition region comprises determining a value of the amplification curve 4 cycles from the first value.

4. The method of claim 1, further comprising normalizing the calculated amplification quality metric.

5. A computer-readable medium encoded with instructions, executable by a processor, the instruction including instructions for:

     determining a first value of a transition region of a PCR amplification curve (500) including an exponential region and the transition region, wherein the first value is a value of the amplification curve at the beginning of the transition region;
     determining a second value of the transition region, wherein the second value is a value of the amplification curve at the end of the transition region;
     calculating an amplification quality metric based on at least the first value and the second value; and
     displaying the amplification quality metric to a user, wherein the beginning of the transition region is the end of the exponential region,
     wherein determining the second value of the transition region comprises determining a value of the amplification curve 2-5 cycles from the first value,
     wherein the beginning of the transition region is determined by computing the peak of the second derivative of the amplification curve (500), and
     wherein calculating the amplification quality metric is taking the difference of the first value and the second value.

6. The computer-readable medium of claim 5, further comprising instructions for filtering the amplification data.

7. The computer-readable medium of claim 5, wherein determining the second value of the transition region comprises determining a value of the amplification curve 4 cycles from the first value.

8. The computer-readable medium of claim 5, further comprising instructions for normalizing the calculated amplification quality metric.

9. A system for providing an amplification quality metric to a user, the system comprising:

a processor (104); and
a memory (106) for storing instructions executable by the processor, the instructions comprising instructions for:

receiving amplification data from a PCR amplification of a sample to generate a PCR amplification curve (500), wherein the amplification curve (500) includes an exponential region and a transition region;
determining a first value of the transition region, wherein the first value is a value of the amplification curve at the beginning of the transition region;
determining a second value of the transition region, wherein the second value is a value of the amplification curve at the end of the transition region;
calculating an amplification quality metric based on at least the first value and the second value; and
displaying the amplification quality metric to a user,
wherein the beginning of the transition region is the end of the exponential region,
wherein determining the second value of the transition region comprises determining a value of the amplification curve 2-5 cycles from the first value,
wherein the beginning of the transition region is determined by computing the peak of the second derivative of the amplification curve (500), and
wherein calculating the amplification quality metric is taking the difference of the first value and the second value.

**Patentansprüche**

1. Computerimplementiertes Verfahren zum Bereitstellen einer Verstärkungsqualitätsmetrik an einen Benutzer, wobei das Verfahren Folgendes umfasst:

Empfangen von Verstärkungsdaten von einer PCR-Verstärkung einer Probe (212) zum Erzeugen einer PCR-Verstärkungskurve (500), wobei die Verstärkungskurve (500) einen Exponentialbereich und einen Übergangsbereich einschließt;
Bestimmen eines ersten Werts des Übergangsbereichs, wobei der erste Wert ein Wert der Verstärkungskurve am Anfang des Übergangsbereichs ist;
Bestimmen eines zweiten Werts des Übergangsbereichs, wobei der zweite Wert ein Wert der Verstärkungskurve am Ende des Übergangsbereichs ist;
Berechnen einer Verstärkungsqualitätsmetrik basierend auf wenigstens dem ersten Wert und dem zweiten Wert; und
Anzeigen der Verstärkungsqualitätsmetrik an einen Benutzer;
wobei der Anfang des Übergangsbereichs das Ende des Exponentialbereichs ist, wobei das Bestimmen des zweiten Werts des Übergangsbereichs das Bestimmen eines Werts der Verstärkungskurve 2-5 Zyklen von dem ersten Wert umfasst,
wobei der Anfang des Übergangsbereichs durch Errechnen der Spitze der zweiten Ableitung der Verstärkungskurve (500) bestimmt wird, und
wobei das Berechnen der Verstärkungsqualitätsmetrik die Differenz des ersten Werts und des zweiten Werts nimmt.

2. Verfahren nach Anspruch 1, das ferner ein Filtern der Verstärkungsdaten umfasst.

3. Verfahren nach Anspruch 1, wobei das Bestimmen des zweiten Werts des Übergangsbereichs das Bestimmen eines Werts der Verstärkungskurve 4 Zyklen von dem ersten Wert umfasst.

4. Verfahren nach Anspruch 1, ferner umfassend ein Normalisieren der berechneten Verstärkungsqualitätsmetrik.

5. Computerlesbares Medium, das mit Anweisungen codiert ist, die von einem Prozessor ausführbar sind, wobei die Anweisung Anweisungen für Folgendes einschließt:

Bestimmen eines ersten Werts eines Übergangsbereichs einer PCR-Verstärkungskurve (500), die einen Exponentialbereich und den Übergangsbereich einschließt, wobei der erste Wert ein Wert der Verstärkungskurve am Anfang des Übergangsbereichs ist;
Bestimmen eines zweiten Werts des Übergangsbereichs, wobei der zweite Wert ein Wert der Verstärkungskurve am Ende des Übergangsbereichs ist;

Berechnen einer Verstärkungsqualitätsmetrik basierend auf wenigstens dem ersten Wert und dem zweiten Wert; und

Anzeigen der Verstärkungsqualitätsmetrik an einen Benutzer, wobei der Anfang des Übergangsbereichs das Ende des Exponentialbereichs ist,

wobei das Bestimmen des zweiten Werts des Übergangsbereichs das Bestimmen eines Werts der Verstärkungskurve 2-5 Zyklen von dem ersten Wert umfasst,

wobei der Anfang des Übergangsbereichs durch Errechnen der Spitze der zweiten Ableitung der Verstärkungskurve (500) bestimmt wird, und

wobei das Berechnen der Verstärkungsqualitätsmetrik die Differenz des ersten Werts und des zweiten Werts nimmt.

6. Computerlesbares Medium nach Anspruch 5, ferner umfassend Anweisungen zum Filtern der Amplifikationsdaten.

7. Computerlesbares Medium nach Anspruch 5, wobei das Bestimmen des zweiten Werts des Übergangsbereichs ein Bestimmen eines Werts der Verstärkungskurve 4 Zyklen von dem ersten Wert umfasst.

8. Computerlesbares Medium nach Anspruch 5, ferner umfassend Anweisungen zum Normalisieren der berechneten Verstärkungsqualitätsmetrik.

9. System zum Bereitstellen einer Verstärkungsqualitätsmetrik an einen Benutzer, wobei das System Folgendes umfasst:

einen Prozessor (104); und

einen Speicher (106) zum Speichern von Anweisungen, die von dem Prozessor ausführbar sind, wobei die Anweisungen Anweisungen für Folgendes umfassen:

Empfangen von Verstärkungsdaten von einer PCR-Verstärkung einer Probe, um eine PCR-Verstärkungskurve (500) zu erzeugen, wobei die Verstärkungskurve (500) einen Exponentialbereich und einen Übergangsbereich einschließt;

Bestimmen eines ersten Werts des Übergangsbereichs, wobei der erste Wert ein Wert der Verstärkungskurve am Anfang des Übergangsbereichs ist;

Bestimmen eines zweiten Werts des Übergangsbereichs, wobei der zweite Wert ein Wert der Verstärkungskurve am Ende des Übergangsbereichs ist;

Berechnen einer Verstärkungsqualitätsmetrik basierend auf wenigstens dem ersten Wert und dem zweiten Wert; und

Anzeigen der Verstärkungsqualitätsmetrik an einen Benutzer,

wobei der Anfang des Übergangsbereichs das Ende des Exponentialbereichs ist, wobei das Bestimmen des zweiten Werts des Übergangsbereichs das Bestimmen eines Werts der Verstärkungskurve 2-5 Zyklen von dem ersten Wert umfasst,

wobei der Anfang des Übergangsbereichs durch Errechnen der Spitze der zweiten Ableitung der Verstärkungskurve (500) bestimmt wird, und

wobei das Berechnen der Verstärkungsqualitätsmetrik die Differenz des ersten Werts und des zweiten Werts nimmt.

## Revendications

1. Procédé mis en oeuvre par ordinateur et destiné fournir une métrique de qualité d'amplification à un utilisateur, le procédé comprenant les opérations suivantes :

recevoir des données d'amplification d'une amplification PCR d'un échantillon (212) pour générer une courbe d'amplification PCR (500), la courbe d'amplification (500) comprenant une région exponentielle et une région de transition ;

déterminer une première valeur de la région de transition, la première valeur étant une valeur de la courbe d'amplification au début de la région de transition ;

déterminer une deuxième valeur de la région de transition, la deuxième valeur étant une valeur de la courbe d'amplification à la fin de la région de transition ;

calculer une métrique de qualité d'amplification basée sur au moins la première valeur et la deuxième valeur ; et

afficher la métrique de qualité d'amplification à destination d'un utilisateur ;

le début de la région de transition étant la fin de la région exponentielle,

la détermination de la deuxième valeur de la région de transition comprenant la détermination d'une valeur de la courbe d'amplification 2 à 5 cycles à partir de la première valeur,

le début de la région de transition étant déterminé par calcul du pic de la dérivée seconde de la courbe d'amplification (500), et

le calcul de la métrique de qualité d'amplification consistant à prendre la différence entre la première valeur et la deuxième valeur.

**2.** Procédé selon la revendication 1, comprenant en outre le filtrage des données d'amplification.

**3.** Procédé selon la revendication 1, la détermination de la deuxième valeur de la région de transition comprenant la détermination d'une valeur de la courbe d'amplification 4 cycles à partir de la première valeur.

**4.** Procédé selon la revendication 1, comprenant en outre la normalisation de la métrique de qualité d'amplification calculée.

**5.** Support lisible par ordinateur, codé avec des instructions, exécutables par un processeur, les instructions comprenant des instructions destinées à :

déterminer une première valeur d'une région de transition d'une courbe d'amplification PCR (500) comprenant une région exponentielle et la région de transition, la première valeur étant une valeur de la courbe d'amplification au début de la région de transition ;

déterminer une deuxième valeur de la région de transition, la deuxième valeur étant une valeur de la courbe d'amplification à la fin de la région de transition ;

calculer une métrique de qualité d'amplification basée sur au moins la première valeur et la deuxième valeur ; et

afficher la métrique de qualité d'amplification à destination d'un utilisateur, le début de la région de transition étant la fin de la région exponentielle,

la détermination de la deuxième valeur de la région de transition comprenant la détermination d'une valeur de la courbe d'amplification 2 à 5 cycles à partir de la première valeur,

le début de la région de transition étant déterminé par calcul du pic de la dérivée seconde de la courbe d'amplification (500), et

le calcul de la métrique de qualité d'amplification consistant à prendre la différence entre la première valeur et la deuxième valeur.

**6.** Support lisible par ordinateur selon la revendication 5, comprenant en outre des instructions destinées à filtrer les données d'amplification.

**7.** Support lisible par ordinateur selon la revendication 5, la détermination de la deuxième valeur de la région de transition comprenant la détermination d'une valeur de la courbe d'amplification 4 cycles à partir de la première valeur.

**8.** Support lisible par ordinateur selon la revendication 5, comprenant en outre des instructions destinées à normaliser la métrique de qualité d'amplification calculée.

**9.** Système destiné à fournir une métrique de qualité d'amplification à un utilisateur, le système comprenant :

un processeur (104) ; et
une mémoire (106) destinée à stocker des instructions exécutables par le processeur, les instructions comprenant des instructions destinées à :

recevoir des données d'amplification d'une amplification PCR d'un échantillon pour générer une courbe d'amplification PCR (500), la courbe d'amplification (500) comprenant une région exponentielle et une région de transition ;

déterminer une première valeur de la région de transition, la première valeur étant une valeur de la courbe d'amplification au début de la région de transition ;

déterminer une deuxième valeur de la région de transition, la deuxième valeur étant une valeur de la courbe d'amplification à la fin de la région de transition ;

calculer une métrique de qualité d'amplification basée sur au moins la première valeur et la deuxième valeur ; et

afficher la métrique de qualité d'amplification à destination d'un utilisateur,

le début de la région de transition étant la fin de la région exponentielle, la détermination de la deuxième valeur de la région de transition comprenant la détermination d'une valeur de la courbe d'amplification 2 à 5 cycles à partir de la première valeur,

le début de la région de transition étant déterminé par calcul du pic de la dérivée seconde de la courbe d'amplification (500), et

le calcul de la métrique de qualité d'amplification consistant à prendre la différence entre la première valeur et la deuxième valeur.

FIG. 1

FIG. 2

**FIG. 3B**

**FIG. 3A**

**FIG. 3C**

FIG. 4

FIG. 5A

FIG. 5B

# OA Data

## Amp Score

### TH = 1.00

**Amp**

| TP | 14625 |
|----|-------|
| FN | 237 |

Sensitivity: 98.41%

**Non-Amp**

| TN | 22153 |
|----|-------|
| FP | 28 |

Specificity: 99.87%

### TH = 1.25

**Amp**

| TP | 12544 |
|----|-------|
| FN | 2318 |

Sensitivity: 84.40%

**Non-Amp**

| TN | 22181 |
|----|-------|
| FP | 0 |

Specificity: 100.00%

**FIG. 6**

OA Non-amp Data Scatter Plot

FIG. 7

OA Amp Data Scatter Plot

Ct Type: Amp

FIG. 8

EP 2 780 853 B1

TaqMan Data

Amp Score

TH = 1.00

TH = 0.7

Amp

| TP | 9335 |
|----|------|
| FN | 225 |

Sensitivity: 97.65%

Amp

| TP | 9529 |
|----|------|
| FN | 31 |

Sensitivity: 99.68%

Non-Amp

| TN | 753 |
|----|-----|
| FP | 1 |

Specificity: 99.87%

Non-Amp

| TN | 723 |
|----|-----|
| FP | 31 |

Specificity: 95.89%

FIG. 9

FIG. 10

FIG. 11